# EUROPEAN PATENT SPECIFICATION

(11) **EP 1 467 696 B1**
(45) Date of publication and mention of the grant of the patent: **08.08.2012**
(21) Application number: 02793021.3
(22) Date of filing: 13.12.2002
(51) Int. Cl.: A61K 8/04, A61K 8/34, A61K 8/73, A61Q 5/06, A61Q 5/12

(54) **Cosmetic or personal care composition**
Kosmetische oder körperpflegende Zusammensetzung
Composition cosmétique ou de soins corporels

(30) Priority: 25.01.2002 GB 0201743
(43) Date of publication of application: 20.10.2004
(73) Proprietor: Unilever N.V., 3013 AL Rotterdam (NL); Unilever PLC, London EC4Y 0DY (GB)
(72) Inventor: EVANS, Jeannette Marcia, Unilever R & D, Sharnbrook, Bedfordshire MK44 1LQ (GB); FRITH, William James, Unilever R & D, Sharnbrook, Bedfordshire MK44 1LQ (GB); LEE, Maria, Unilever R & D Port Sunlight, Wirral, Merseyside CH63 3JW (GB); WOLF, Bettina, Unilever R & D, Sharnbrook, Bedfordshire MK44 1LQ (GB)
(74) Representative: Newbould, Frazer Anthony
(86) International application number: PCT/EP2002/014286
(87) International publication number: WO 2003/061607

(56) References cited:
- EP-A- 0 574 973
- EP-A- 1 285 588
- EP-A1- 0 355 908
- WO-A-02/36086
- WO-A-95/12988
- WO-A-98/08601
- PATENT ABSTRACTS OF JAPAN vol. 2000, no. 12, 3 January 2001 (2001-01-03) & JP 2000 239147 A (NARIS COSMETICS CO LTD), 5 September 2000 (2000-09-05)
- WOLF H ET AL: "INFLUENCE OF GELATION ON PARTICLE SHAPE IN SHEARED BIOPOLYMER BLENDS" JOURNAL OF RHEOLOGY, AMERICAN INSTITUTE OF PHYSICS, EASTON, PA, US, vol. 45, no. 5, September 2001 (2001-09), pages 1141-1157, XP001038774 ISSN: 0097-0360

## Description

This invention relates to a cosmetic or personal care composition, to a method of treating hair, to the use of certain materials in cosmetic and personal care compositions, and to processes for producing the compositions.

Cosmetic and personal care compositions are often required to have certain textural attributes. For example, shampoo and hair conditioner compositions are generally required to have textural properties, in addition to their ability to clean and/or condition hair, in order to be acceptable to the consumer. It is for this reason that many commercial shampoo and hair conditioner compositions contain a thickener or viscosity modifier to increase the viscosity of the composition and provide a product that is acceptable to the consumer.

Other cosmetic and personal care compositions rely on the rheological properties of the composition not only for textural benefits but also to enhance the stability of the dispensed product and to enhance the deposition and/or retention of the product at the site of use. For example, hair styling products such as gels and aerosol mousses need to be stable when dispensed and anti-dandruff products (such as shampoos and scalp lotions) need to be formulated such that the anti-dandruff active agent is effectively deposited on the scalp.

It is known that gel particles can be incorporated into hair treatment compositions. Our copending international (PCT) application no PCT/EP01/11775, filed 10 October 2001, discloses the use of shear gels in hair treatment compositions. The shear gel particles are generally substantially non-spherical and have low aspect ratios.

WO98/08601 describes aqueous compositions such as liquid personal cleansers containing large hydrogel particles formed by two different water soluble polymers. The hydrogel particles trap water insoluble benefit agents in a network formed by these two polymers. The system is not a shear gel since it is prepared by first forming elongated polymer gel noodles which after gel formation are subsequently cut/broken into the desired gel particle size.

There remains a need for cosmetic and personal care compositions with novel and/or well-defined textural properties.

There is also a need for cosmetic and personal care compositions, which have effective deposition of active agents.

There has recently been interest in the specific and targeted delivery of hair benefit agents, such as oils and silicones, to the hair fibre. The delivery is ideally specific, so that the oil/silicone is delivered in a greater amount than any undesirable components of the shampoo and targeted so that more oil/silicone is deposited on the hair tips than at the hair roots. Therefore, there is also a need for hair treatment compositions, which have specific and/or targeted delivery of hair benefit agents.

The formation of gelled fibres using biopolymers can be achieved by gelling the droplet phase of a liquid two-phase mixture in a shear field. The formation of these gelled fibres is described in Wolf et al, Food Hydrocolloids, 14 (2000), 217-225 and Wolf et al, Rheol Acta (2001), 40:238-247. No application of the gelled fibres is described in either of the two documents.

The formation of gel-like fibres is also described in Tolstoguzov, Food Proteins and their Applications, 1997, Ed Damodaran et al, Marcel Dekker, Inc, New York and in Suchkov et al, Nahrung, 32(7), 661-8. Again, there is no mention of cosmetic or personal care compositions.

The present invention relates to cosmetic and personal care compositions having unexpected textural attributes that are desirable for the consumer. The compositions may also exhibit the property of effective deposition onto hair fibres. Furthermore, in certain embodiments, the compositions may exhibit desirable properties in relation to the delivery of hair benefit agents.

According to the present invention, in a first aspect, there is provided a cosmetic or personal care composition comprising:
gelled particles, wherein the gelled particles comprise water and at least 50% by volume of the particles have an aspect ratio of at least 5 and a maximum width of 40 µm; and
at least one cosmetically acceptable material selected from surfactants, hair conditioning agents, perfumes or fragrances and hair styling polymers.

In a second aspect, the invention provides a method of treating hair comprising applying to the hair a composition comprising gelled particles comprising water, wherein at least 50% by volume of the particles have an aspect ratio of at least 5 and a maximum width of 40 µm.

A further aspect of the invention is the use of gelled particles comprising water wherein at least 50% by volume of the particles have an aspect ratio of at least 5 and a maximum width of 40 µm in a cosmetic or personal care composition.

In another aspect, the invention provides a process for producing a cosmetic or personal care composition according to the invention which comprises forming the gelled particles and then bringing the gelled particles into contact with the at least one cosmetically acceptable material.

Yet another aspect of the invention is process for producing a cosmetic or personal care composition according to the invention that comprises at least one surfactant, which process comprises forming the gelled particles in the presence of the one or more surfactants.

A further aspect of the invention is the use of gelled particles comprising water wherein at least 50% by volume of the particles have an aspect ratio of at least 5 and a maximum width of 40 µm for the delivery of at least one hair benefit agent to the hair.

An even further aspect of the invention is the use of gelled particles comprising water wherein at least 50% by volume of the particles have an aspect ratio of at least 5 and a maximum width of 40 µm for providing textural properties (such as a feeling of creaminess) to a cosmetic or personal care composition.

The present invention involves the use of gelled particles which comprise water and in which at least 50% by volume of the particles have an aspect ratio of at least 5 and a maximum width of 40 µm, in cosmetic and personal care compositions. Such gelled particles are also termed gelled fibres herein.

The gelled particles of the invention contain at least 50% by volume of particles having an aspect ratio of at least 5 and a maximum width of 40 µm. More preferably, at least 20% by volume of the particles have an aspect ratio of at least 10 and a maximum width of 20 µm. Even more preferably, at least 5% by volume of the particles have an aspect ratio of at least 50 and a maximum width of 2 µm. Suitably, greater than 90% by volume of the particles, and preferably all of the particles, have a minimum width of 0.5 µm.

The gelled particles comprise water, typically in an amount of from 1% to 99%, such as from 30% to 98%, for example 50% to 98%, by weight of the gelled particles. The gelled particles also comprise at least one gelling polymer that is responsible for the gelled structure of the particles. The gelling polymer can, for example, be a protein or a polysaccharide. Suitable gelling polymers include kappa-carrageenan, gellan, gelatin, alginate, and mixtures thereof. Preferred are gellan, kappa-carrageenan and mixtures thereof, with kappa-carrageenan being particularly preferred.

The gelling polymer may form a gel alone or may require the addition of another substance, such as, for example, another polymer or a metal ion, for example an alkali metal or an alkaline earth metal, in order for gelation to take place. Conditions under which gelling polymers can be caused to form a gel are well known to those skilled in the art.

The gelled fibres of the invention are capable of imparting beneficial textural properties to a composition of the invention. For example, compositions containing gelled fibres according to the invention may have an interesting feel or sensation when applied to the hair and/or the scalp, including wet hair, as well as good textural properties when present on the hands of the user of the product.

The gelled fibres of the invention are generally particles that range in shape from ellipsoidal to highly elongated in one direction. When present in compositions of the invention, the gelled fibres may be anisotropically arranged or isotropically arranged. Typically, each of the gelled fibres will be extended to some extent in a certain direction in compositions of the invention.

Gelation to form the gelled fibres of the invention can be carried out in any suitable way. The gelation treatment is preferably selected from the group comprising temperature treatment, chemical gelation or crystallisation. The gelation method that is selected in any given case depends on the ingredients of the final composition.

Gelation by temperature treatment is preferred if the gelling polymer is dependent on temperature for its gel formation. Examples of such gelling agents include gelatin, which gels at a temperature of below about 40°C, agar which forms a gel at a temperature of below about 45°C and carrageenan or gellan, for which gelation temperature is dependent on salt type and concentration, as described in Handbook of Hydrocolloids, Ed Phillips and Williams, CRC Press, the contents of which are incorporated by reference herein. Proteins that gel or form a network on heat treatment are also suitable for the formation of gelled fibres.

It will be appreciated that the exact gelling temperature for the gelling polymer will be determined by, amongst other properties, quality, purity, concentration, solvent properties (such as added solutes and co-solvents) and pH.

Alternatively, the gelling polymer may form a gel by interaction with another component. Typically, such gelling polymers are those polymers which, after being dispersed in another phase, such as a liquid, will set to a gel when allowed to interact with a supplementary active component. The active component is typically a metal cation. Alternatively, the active component may cause the polymer to gel as a result of a chemical reaction such as oxidation. A further example of a gelling polymer of this type is the class of polymers that gel upon a change in pH, for example polymers, which set or precipitate at a pH below their isoelectric point.

The use of polymers that require a metal cation for gelation is typically preferred. The gelation can be effected by combining the gelling polymer with a salt having a suitable cation. The cation is preferably selected from calcium and potassium ions. Potassium ions are used to cause, for example, gelation of kappa-carrageenan.

Formation of the gelled fibres may be caused to take place as a result of phase separation. The phase separation results in the formation of a gelled phase i.e., the gelled fibres. At least two polymers are required for phase separation to take place. The phase separation may be segregative phase separation, in which formation of the gelled phase occurs as a result of a net repulsion between the at least two polymers. Alternatively, the phase separation may be associative phase separation in which the gelled phase is formed as a result of a net attraction or association between the at least two polymers. Segregative phase separation is preferred for forming gelled fibres. Segregative phase separation is described in Tolstoguzov, Food Proteins and their Applications, 1997, Ed Damodaran et al, Marcel Dekker, Inc, New York, the contents of which are incorporated by reference herein. Examples of pairs of polymers that undergo segregative phase separation include: ovalbumin and soybean globulin; casein and soybean globulin; gelatin and soybean globulin; amylopectin and casein; sodium alginate and casein; methyl cellulose and pectin; maltodextrin and gelatin; bovine serum albumin (BSA) and carboxymethylcellulose (CMC) at pH 5.3; anionic polysaccharides (high methoxy (HM) pectins, alginate, CMC) and denatured whey proteins; neutral polysaccharides (dextran, methyl cellulose (MC), maltodextrin) and native whey proteins (pH5-7); dextran or hydroxyethylcellulose (HEC) and BSA, gamma-globulin or lysozyme; and casein with kappa-carrageenan.

Concentrations of the at least two polymers required to form a gel by segregative phase separation can be determined by the skilled person, with reference to Tolstoguzov as referred to above, for example. At least one of the at least two polymers will be a gelling polymer.

The formation of gelled fibres typically involves subjecting the gelled phase, before and/or during gel formation, to forces that cause elongation of the gel particles. Typically, the gelled phase is subjected to shear forces during its formation as described, for example, in Wolf et al, Food Hydrocolloids, 14(2000), 217-225 and Wolf et al, Rheol Acta (2001), 40:238-247.

In one embodiment of the process for producing a composition according to the invention, the gelled particles are formed and are then brought into contact with the at least one cosmetically acceptable material. Preferably, the gelled particles are formed by a method comprising the steps of
a) mixing two aqueous phases each comprising a polymer, wherein at least one of the polymers is a gelling polymer;
b) treating the mixture according to a) such that one of the phases is present in the form of droplets in the second phase;
c) subjecting the mixture to shear flow; and
d) causing the polymer to gel during or after step (c).

Step (c) is preferably carried out in a flow through device, or an array of parallel flow through devices that are adapted to subject the mixture to shear flow. By the term flow through device, we mean a device that allows application of the appropriate shear flow to the mixture, such as, for example, a pipe or a plate heat exchanger. The flow devices are typically extended (ie, elongated) in the flow direction and may be cylindrical, although cross-sections other than circular are possible; the main requirement is that the mixture is subjected to shear forces as it is passed through the flow devices. Commercially available process apparatus fulfilling the above described requirements could be non-contact type tubular and plate heat exchangers as described in Introduction to Food Engineering 3rd Edition, Singh and Heldmann, Academic Press, San Diego, 2001. Other methods suitable for forming the gelled particles include all types of shear rheometers as, e.g., described in Rheology: Principles, measurements, and applications, Macosko, VCH Publisher Inc., Weinheim, 1994, and flow processes based on the same types of flow geometries.

Steps (a) and (b) may be carried out in a pre-mix tank. Preferably, step (a) is simplified by mixing the two polymers in powder form and adding them in one step to a common aqueous solvent. Depending on the polymer combinations used, the mixture in powder form may additionally comprise a salt such as sodium chloride or potassium chloride.

The flow rate in the flow through device or devices is typically from 0.1 to 25 ml/min and the wall shear stress is from 15 to 800Pa. For the definition of wall shear stress, reference is made to textbooks on rheology such as, for example, Rheology: Principles, Measurements and Applications, C W Macosko, VCH Publishers, Inc, 1994.

Gelation can be caused to take place in any of the ways described above. Preferably, gelation involves segregative phase separation to form a gelling phase comprising kappa-carrageenan, which gels in the presence of potassium ions present in the composition.

The gelled fibres may be added to the at least one cosmetically acceptable material directly after step (d). Alternatively, the gelled fibres may be separated from all or a part of the mixture formed from step (d), before being brought into contact with the at least one cosmetically acceptable material. Separation of the gelled fibres from the mixture formed in step (d) can be accomplished by physical separation eg, centrifugation. Optionally, after separation, the gelled fibres can be rinsed (eg, with water) to remove all or a part of the remaining mixture formed in step (d) before a further separation step.

The gelled particles are brought into contact with the at least one cosmetically acceptable material, preferably with a step of mixing the particles with the material. The material may be added to the gelled particles or the gelled particles may be added to the material or other methods may be employed to cause the particles and the material to come into contact with each other.

In another embodiment, the process of the invention produces a composition of the invention that comprises a surfactant. The process comprises forming the gelled particles in the presence of the surfactant. Surprisingly, it is possible to produce the gelled particles even in the presence of a surfactant, which, it might reasonably have been expected, could cause the break up of the gelled particles or could prevent them from being formed. This embodiment of the invention may comprise steps (a) to (d) set out above with the surfactant being present in at least one of the aqueous phases of step (a). The process has the advantage that there is no need to bring the gelled particles into contact with the surfactant in a subsequent processing step. Therefore, the final composition can be produced by forming the gelled particles as a final processing step or by adding minor ingredients to a pre-formed mixture of gelled particles and surfactant.

The gelled particles of the invention, in one embodiment, comprise a hair benefit agent. The hair benefit agent may be encapsulated within the gelled particles, associated with the exterior or interior of the gelled particles or otherwise retained by the gelled particles such that the hair benefit agent is delivered to the hair together with the gelled particles.

Hair benefit agents may be single compounds or materials or mixtures of different compounds or materials. The or each hair benefit agent is capable of imparting beneficial properties when used in a hair treatment product, including properties beneficial or desirable on the hair and benefits for the scalp. Benefits include, for example, hair conditioning, hair colouring, hair styling and antidandruff benefits. Hair conditioning benefits are particularly preferred.

The or each hair benefit agent can be hydrophilic or hydrophobic. When the hair benefit agent is hydrophilic, it may form a part of the gelled phase. When the hair benefit agent is hydrophobic, it may form a separate phase within and/or at the surface of the gelled phase, for example in the form of droplets.

The one or more hair benefit agents are present in a sufficient amount to perform the intended function, typically in an amount of about 0.1% to about 30% by weight of the gelled particles.

Examples of suitable hair benefit agents include, but are not limited to, hair and skin conditioners, hair and skin cleansers, hair fixatives (including hair styling polymers), hair dyes, hair growth promoters, deodorants, skin care compounds, permanent wave compounds, hair relaxers, hair straighteners, antibacterial compounds, antifungal compounds, anti-inflammatory compounds, topical anesthetics, sunscreens and other cosmetic and medicinal topically-effective compounds.

Hydrophobic oils, such as hydrocarbon oils, esters containing from 8 to 30 carbon atoms and particularly silicone oils, are preferred hair benefit agents.

Compositions of the invention having gelled fibres comprising a hair benefit agent can be formed, for example, by incorporating an emulsion, suspension, dispersion or solution of the hair benefit agent into the mixture before gelling of the polymer takes place, in the processes of the invention that are described above.

Without wishing to be bound by theory, it is believed that the shape and flexibility of the gelled particles of the invention mean that they can: entangle amongst the hair fibres and therefore have increased resistance to being rinsed off the hair; and be more likely to be washed down and therefore deposit on the hair tips rather than the hair roots. On drying the hair, the gelled fibres can dry out leaving the oil on the hair. As the concentration of polymer in the gelled particles can be low, any residue from the fibres may be small relative to the amount of hair benefit agent.

In the compositions of the invention, the gelled particles are preferably present in an amount of from 0.1% to 60%, preferably from 1% to 50%, even more preferably from 1% to 30%, by volume of the composition, based on the total composition. Typically, this equates to an amount of gelled fibres of from about 0.1% to about 50% by weight, based on weight of total composition.

The composition of the invention is a cosmetic or personal care composition, examples of which include personal wash compositions such as soaps and shower gels. Preferably, the composition is a hair treatment composition and the compositions will typically be packaged and labelled as such.

Hair treatment compositions of the invention include, for example, shampoos, conditioners, hair styling products, tonics and lotions.

Shampoo compositions of the invention comprise from 1% to 50%, preferably from 1% to 30%, more preferably from 5% to 30%, by weight of one or more surfactants.

Conditioners, also termed hair conditioning compositions, comprise from 0.1% to 30% preferably from 1% to 20%, more preferably from 2% to 15 %, by weight of one or more hair conditioning agents.

Hair styling compositions comprise from 0.1% to 10%, preferably from 1% to 10%, more preferably from 2% to 8% by weight of one or more hair styling polymers.

Compositions of the invention can be leave on or rinse off compositions. Rinse off compositions are intended to be rinsed from the hair after use, although a minor proportion of the composition, including at least some of the particles, will remain on the hair after rinsing. Leave on products are applied to the hair and need not be rinsed off the hair after this application.

Compositions of the present invention can be formulated into a wide variety of product types, including mousses, gels, lotions, tonics, sprays, shampoos, conditioners, rinses, and the like. Compositions of the invention comprise, in addition to the particles and the cosmetically acceptable material, a cosmetically acceptable diluent or carrier.

Compositions of the present invention can comprise a carrier, or a mixture of such carriers, which are suitable for application to the hair. The carriers are present at from about 0.5% to about 99.5%, preferably from about 5.0% to about 99.5%, more preferably from about 10.0% to about 98.0%, by weight of the composition. As used herein, the phrase "suitable for application to hair" means that the carrier does not damage or negatively affect the aesthetics of hair or cause irritation to the underlying skin.

Carriers suitable for use with hair care compositions of the present invention include, for example, those used in the formulation of hair sprays, mousses, tonics, gels, shampoos, conditioners, and rinses. The choice of appropriate carrier will also depend on the particular formulation, and whether the product formulated is meant to be left on the surface to which it is applied (e.g., hair spray, mousse, tonic, or gel) or rinsed off after use (e.g., shampoo, conditioner or rinse).

The carriers used herein can include a wide range of components conventionally used in hair care compositions. The carriers can contain a solvent to disperse the particles being used, with water, the C₁-C₆ alcohols, lower alkyl acetate and mixtures thereof being preferred. The carriers can also contain a wide variety of additional materials such as acetone, hydrocarbons (such as isobutane, hexane, decene), halogenated hydrocarbons (such as Freons) and volatile silicones such as cyclomethicone.

Where the hair care compositions are conditioners and rinses, the carrier can include a wide variety of conditioning materials. Where the hair care compositions are shampoos, the carrier can include, for example, surfactants, suspending agents, and thickeners. Hair styling creams or gels also typically contain a structurant or thickener, typically in an amount of from 0.01% to 10% by weight.

The carrier can be in a wide variety of forms. For example, emulsion carriers, including oil-in-water, water-in-oil, water-in-oil-in-water, and oil-in-water-in-silicone emulsions, are useful herein. These emulsions can cover a broad range of viscosities, e.g., from about 100 cps to about 200,000 cps. These emulsions can also be delivered in the form of sprays using either mechanical pump containers or pressurised aerosol containers using conventional propellants. These carriers can also be delivered in the form of a mousse. Other suitable topical carriers include anhydrous liquid solvents such as oils, alcohols, and silicones (e.g., mineral oil, ethanol, isopropanol, dimethicone, cyclomethicone, and the like); aqueous-based single phase liquid solvents (e.g., hydro-alcoholic solvent systems); and thickened versions of these anhydrous and aqueous-based single phase solvents (e.g., where the viscosity of the solvent has been increased to form a solid or semi-solid by the addition of appropriate gums, resins, waxes, polymers, salts, and the like).

Compositions of the invention typically contain up to 50% by weight of the composition of the particles of the invention, preferably from 0.01% to 50% by weight, more preferably from 0.05% to 30% by weight, such as 1% to 20% by weight.

The following describes components that may be contained in compositions of the invention separate from the particles ie, in a separate phase from the particles and generally in the phase in which the particles are dispersed or suspended.

### Shampoo and hair conditioning compositions

Examples of rinse off compositions of the invention are shampoo compositions and hair conditioning compositions.

Shampoo compositions of the invention comprise, in addition to the particles, at least one surfactant which provides a deterging benefit. The deterging surfactant is preferably selected from anionic, nonionic, amphoteric and zwitterionic surfactants, and mixtures thereof.

Suitable anionic surfactants include the alkyl sulphates, alkyl ether sulphates, alkaryl sulphonates, alkanoyl isethionates, alkyl succinates, alkyl sulphosuccinates, N-alkoyl sarcosinates, alkyl phosphates, alkyl ether phosphates, alkyl ether carboxylates, and alpha-olefin sulphonates, especially their sodium, magnesium ammonium and mono-, di- and triethanolamine salts.

The most preferred anionic surfactants are sodium lauryl sulphate, triethanolamine lauryl sulphate, triethanolamine monolauryl phosphate, sodium lauryl ether sulphate 1EO, 2EO and 3EO, ammonium lauryl sulphate and ammonium lauryl ether sulphate 1EO, 2EO and 3EO.

Nonionic surfactants suitable for use in compositions of the invention may include condensation products of aliphatic (C₈-C₁₈) primary or secondary linear or branched chain alcohols or phenols with alkylene oxides, usually ethylene oxide and generally having from 6 to 30 ethylene oxide groups. Other suitable nonionics include mono- or di-alkyl alkanolamides. Examples include coco mono- or di- ethanolamide and coco mono-isopropanolamide.

Further nonionic surfactants which can be included in shampoos for the invention are the alkyl polyglycosides (APGs). Typically, the APG is one, which comprises an alkyl group connected (optionally via a bridging group) to a block of one or more glycosyl groups. Preferred APGs are defined by the following formula:

RO - (G)n

wherein R is a branched or straight chain alkyl group which may be saturated or unsaturated and G is a saccharide group. Suitable alkyl polyglycosides for use in the invention are commercially available and include for example those materials identified as: Oramix NS10 ex Seppic; Plantaren 1200 and Plantaren 2000 ex Henkel.

Amphoteric and zwitterionic surfactants suitable for use in compositions of the invention may include alkyl amine oxides, alkyl betaines, alkyl amidopropyl betaines, alkyl sulphobetaines (sultaines), alkyl glycinates, alkyl carboxyglycinates, alkyl amphopropionates, alkylamphoglycinates alkyl amidopropyl hydroxysultaines, acyl taurates and acyl glutamates, wherein the alkyl and acyl groups have from 8 to 19 carbon atoms. Examples include lauryl amine oxide, cocodimethyl sulphopropyl betaine and preferably lauryl betaine, cocamidopropyl betaine and sodium cocamphopropionate.

The surfactants are present in shampoo compositions of the invention in an amount of from 1 to 50% by weight of the composition, preferably from 1 to 30% by weight, more preferably from 5 to 30% by weight.

Compositions in accordance with the invention may also take the form of hair conditioning compositions, which may be rinse off or leave-on hair conditioning compositions or so-called 2 in 1 compositions containing shampoo and conditioner. The conditioning compositions preferably comprise, in addition to the particles, one or more cationic surfactants. The use of cationic surfactants is especially preferred, because these ingredients are capable of providing conditioning benefits to hair.

Examples of cationic surfactants include the quaternary ammonium compounds mentioned hereinbefore as optional components of the hydrophilic phase. These include: quaternary ammonium hydroxides, e.g., tetramethylammonium hydroxide, alkyltrimethylammonium hydroxides wherein the alkyl group has from about 8 to 22 carbon atoms, for example octyltrimethylammonium hydroxide, dodecyltrimethyl ammonium hydroxide, hexadecyltrimethylammonium hydroxide, cetyltrimethylammonium hydroxide, octyldimethylbenzylammonium hydroxide, decyldimethyl-benzylammonium hydroxide, stearyldimethylbenzylammonium hydroxide, didodecyldimethylammonium hydroxide, dioctadecyldimethylammonium hydroxide, tallow trimethylammonium hydroxide, cocotrimethylammonium hydroxide, and the corresponding salts thereof containing anions other than hydroxide, e.g., chlorides, Cetylpyridinium hydroxide or salts thereof (e.g., chloride), Quaternium -5, Quaternium -31, Quaternium -18, and mixtures thereof.

In hair conditioning compositions according to the invention, the level of cationic surfactant is preferably from 0.01 to 10%, more preferably 0.05 to 5%, most preferably 0.1 to 2% by weight of the composition.

Hair conditioning and shampoo compositions of the invention may both also contain one or more additional conditioning agents, preferably selected from silicones, protein hydrolysates and quaternised protein hydrolysates and other materials which are known in the art as having desirable hair conditioning properties.

Silicones are the most preferred conditioning agents. The silicones are preferably in the form of liquid droplets, typically dispersed in compositions of the invention, preferably in an amount of from 0.01% to 5% by weight of the composition, more preferably from 0.1% to 5% by weight

Suitable silicones include volatile and non-volatile silicones, such as for example polyalkylsiloxanes, polyalkylaryl siloxanes, siloxane gums and resins, cyclomethicones, aminofunctional silicones, quaternary silicones and mixtures thereof. Silicone oil is a particularly preferred conditioning agent for hair. The silicone may be in the form of a low viscosity oil which may contain a high viscosity oil or gum in solution. Alternatively, the high viscosity material may be in the form of an emulsion in water. The emulsion may be of high viscosity oil or of a solution of gum in a lower viscosity oil. The particle size of the oil phase may be anywhere in the range from 30 nanometres to up to 20 microns average size.

The silicone oil may suitably be a polydimethylsiloxane with an average particle size of less than 20 microns and preferably less than 2 microns. Small particle size enables a more uniform distribution of silicone conditioning agent for the same concentration of silicone in the composition. Advantageously, a silicone with a viscosity in the range 1-20 million cst is used. The silicone can be cross-linked.

Preferred silicones include polydimethylsiloxanes (of CTFA designation dimethicone) and hydroxylated polydimethylsiloxanes (of CTFA designation dimethiconol). Silicones of the above types are widely available commercially, for example as DC-1784 and DCX2-1391, both ex Dow Corning.

Suitable protein hydrolysates include lauryl dimonium hydroxy propylamino hydrolysed animal protein, available commercially under the trade name LAMEQUAT L, and hydrolysed keratin containing sulphur-bearing amino acids, available commercially under the trade name CROQUAT WKP.

In accordance with the invention, the hair shampoo and/or conditioner composition may also comprise a polymeric watersoluble cationic polymer as a conditioning agent.

The cationic polymer may be present at levels of from 0.01 to 5%, preferably from about 0.05 to 1%, more preferably from about 0.08% to about 0.5% by weight.

Synthetic or naturally derived polymers having a quaternised nitrogen atom are useful. The molecular weight of the polymer (in g/mol) will generally be between 5 000 and 10 000 000, typically at least 10 000 and preferably in the range 100 000 to about 2 000 000.

Representative synthetic quaternised polymers include, for example: cationic copolymers of 1-vinyl-2-pyrrolidine and 1-vinyl-3-methyl-imidazolium salt (e.g., chloride salt) (referred to in the industry by the Cosmetic, Toiletry, and Fragrance Association, "CTFA", as Polyquaternium-16); copolymers of 1-vinyl-2-pyrrolidine and dimethylaminoethyl methacrylate (referred to in the industry by CTFA as Polyquaternium-11); cationic diallyl quaternary ammonium-containing polymers including, for example, dimethyldiallylammonium chloride homopolymer (referred to in the industry (CTFA) as Polyquaternium 6); mineral acid salts of amino-alkyl esters of homo-and co-polymers of unsaturated carboxylic acids having from 3 to 5 carbon atoms, as described in U.S. Patent 4,009,256; and cationic polyacrylamides as described in WO95/22311.

Representative naturally-derived quaternised polymers include quaternised cellulosic compounds and cationic guar gum derivatives, such as guar hydroxypropyltrimonium chloride. Examples are JAGUAR C-13S, JAGUAR C-15, and JAGUAR-C17, commercially available from Meyhall in their JAGUAR (trademark) series.

Suitable cationic polyacrylamides are described in WO 95/22311 whose contents are incorporated herein by reference.

The compositions may further comprise from 0.1 to 5 % of a suspending agent. Examples are polyacrylic acids, cross linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearates, alkanolamides of fatty acids having from 16 to 22 carbon atoms and mixtures thereof. Polyacrylic acid is available commercially as Carbopol 420, Carbopol 488 or Carbopol 493. Polymers of acrylic acid cross-linked with a polyfunctional agent may also be used, they are available commercially as Carbopol 910, Carbopol 934, Carbopol 940, Carbopol 941 and Carbopol 980. An example of a suitable copolymer of a carboxylic acid containing a monomer and acrylic acid esters is Carbopol 1342. All Carbopol materials are available from Goodrich and Carbopol is a trade mark. A further suitable suspending agent is dihydrogenated tallow phthalic acid amide (available from Stepan under the trademark Stepan TAB-2).

Suitable cross linked polymers of acrylic acid and acrylate esters are Pemulen TR1 or Pemulen TR2. A suitable heteropolysaccharide gum is xanthan gum, for example that available as Kelzan mu.

Another ingredient that may advantageously be incorporated into shampoo and/or conditioning compositions of the invention is a fatty alcohol material. The use of these materials is especially preferred in conditioning compositions of the invention, in particular conditioning compositions which comprise one or more cationic surfactant materials. The combined use of fatty alcohol materials and cationic surfactants in conditioning compositions is believed to be especially advantageous, because this leads to the formation of a lamellar phase, wherein the cationic surfactant is dispersed.

Preferred fatty alcohols comprise from 8 to 22 carbon atoms, more preferably 16 to 20. Examples of preferred fatty alcohols are cetyl alcohol and stearyl alcohol. The use of these materials is also advantageous in that they contribute to the overall conditioning properties of compositions of the invention.

The level of fatty alcohol materials is conveniently from 0.01 to 10%, preferably from 0.1 to 5% by weight of the composition. The weight ratio of cationic surfactant to fatty alcohol is preferably from 10:1 to 1:10, more preferably from 4:1 to 1:8, most preferably from 1:1 to 1:4.

A further ingredient that may be desirably included in the shampoo and/or conditioning compositions is a pearlescent material. Suitable pearlescent materials include ethylene glycol distearate, ethylene glycol monostearate, guanine and titanium dioxide coated micas, bismuth oxychloride, and stearic monoethanol amide. The level of pearlescent material present in the composition is generally 0.1% to 5%, preferably from 0.3% to 3% by weight of the composition.

The compositions of the invention may optionally comprise an antimicrobial agent. The antimicrobial agent may be a single compound or a mixture of two or more compounds. The antimicrobial agent may, for example, be in solid particulate form or dissolved in compositions of the invention.

The antimicrobial agent is typically present in compositions of the invention in an amount of from 0.01% to 5% by weight, preferably from 0.1% to 2% by weight.

Preferably, soluble antimicrobial agents are selected from climbazole, ketoconazole, octapirox and mixtures thereof. More preferably, the antimicrobial agent is climbazole. These antimicrobial agents will typically be in solution in compositions of the invention.

The preferred solid antimicrobial agents are metal pyrithiones, particularly zinc pyrithione (ZnPTO) which, on account of its relative insolubility in aqueous systems, is generally used in hair treatment compositions as a particulate dispersion. The zinc pyrithione may be used in any particle form including, for example, crystalline forms such as platelets and needles and amorphous, regularly or irregularly shaped particles. If zinc pyrithione is present in the composition, a suspending agent is preferably used to prevent or inhibit the settling of the particles out of the composition. The average particle diameter of the zinc pyrithione particles (ie, their maximum dimension) is typically from about 0.2 to about 50 µm, preferably from about 0.4 to about 10 µm. Particle size can be measured using a Malvern Mastersizer (Malvern Instruments, Malvern, UK).

Antimicrobial agents typically display a minimum inhibitory concentration of about 50 mg/ml or less against *Malassezia.*

The shampoo and/or conditioner compositions of the invention are preferably aqueous based. The compositions suitably comprise water in amount of from about 20 to about 99% by weight of the total composition.

The shampoo and conditioner compositions of the present invention may also contain other ingredients conventionally used in the art such as diluents, sequestrants, thickeners, carriers, antioxidants, proteins, polypeptides, preservatives, moisturising agents, solvents, perfumes, enzymes and polymers.

### Leave on products

When the hair care composition is a leave on product such as a hair spray, tonic, gel, or mousse the preferred solvents include water, ethanol, volatile silicone derivatives, and mixtures thereof. The solvents used in such mixtures may be miscible or immiscible with each other. Mousses and aerosol hair sprays can also utilise any of the conventional propellants to deliver the material as a foam (in the case of a mousse) or as a fine, uniform spray (in the case of an aerosol hair spray). Examples of suitable propellants include materials such as trichlorofluoromethane, dichlorodifluoromethane, difluoroethane, dimethylether, propane, n-butane or isobutane. A tonic or hair spray product having a low viscosity may also utilise an emulsifying agent. Examples of suitable emulsifying agents include nonionic, cationic, anionic surfactants, or mixtures thereof. If such an emulsifying agent is used, it is preferably present at a level of from about 0.01% to about 7.5% by weight based on total weight of the composition. The level of propellant can be adjusted as desired but is generally from about 3% to about 30% by weight based on total weight for mousse compositions and from about 15% to about 50% by weight based on total weight for aerosol hair spray compositions.

Suitable spray containers are well known in the art and include conventional, non-aerosol pump sprays i.e., "atomisers", aerosol containers or cans having propellant, as described above, and also pump aerosol containers utilising compressed air as the propellant.

A wide variety of additional components can be employed in leave on compositions, such as hair styling compositions, according to the present invention. Examples include the following:
- hair styling polymers for hair styling compositions such as hair sprays, gels, and mousses. Hair styling polymers are well known articles of commerce and many such polymers are available commercially which contain moieties, which render the polymers cationic, anionic, amphoteric or nonionic in nature. The polymers may be synthetic or naturally derived.

The amount of the polymer may range from 0.5 to 10%, preferably 0.75 to 6% by weight based on total weight of the composition.

Examples of anionic hair styling polymers are:
copolymers of vinyl acetate and crotonic acid;
terpolymers of vinyl acetate, crotonic acid and a vinyl ester of an alpha-branched saturated aliphatic monocarboxylic acid such as vinyl neodecanoate;
copolymers of methyl vinyl ether and maleic anhydride (molar ratio about 1:1) wherein such copolymers are 50% esterified with a saturated alcohol containing from 1 to 4 carbon atoms such as ethanol or butanol;
acrylic copolymers containing acrylic acid or methacrylic acid as the anionic radical-containing moiety with other monomers such as: esters of acrylic or methacrylic acid with one or more saturated alcohols having from 1 to 22 carbon atoms (such as methyl methacrylate, ethyl acrylate, ethyl methacrylate, n-butyl acrylate, t-butyl acrylate, t-butyl methacrylate, n-butyl methacrylate, n-hexyl acrylate, n-octyl acrylate, lauryl methacrylate and behenyl acrylate); glycols having from 1 to 6 carbon atoms (such as hydroxypropyl methacrylate and hydroxyethyl acrylate); styrene; vinyl caprolactam; vinyl acetate; acrylamide; alkyl acrylamides and methacrylamides having 1 to 8 carbon atoms in the alkyl group (such as methacrylamide, t-butyl acrylamide and n-octyl acrylamide); and other compatible unsaturated monomers.

The polymer may also contain grafted silicone, such as polydimethylsiloxane.

Specific examples of suitable anionic hair styling polymers are:
RESYN® 28-2930 available from National Starch (vinyl acetate/crotonic acid/vinyl neodecanoate copolymer);
ULTRAHOLD® 8 available from BASF (CTFA designation Acrylates/acrylamide copolymer);
the GANTREZ®ES series available from ISP corporation (esterified copolymers of methyl vinyl ether and maleic anhydride).

Other suitable anionic hair styling polymers include carboxylated polyurethanes. Carboxylated polyurethane resins are linear, hydroxyl-terminated copolymers having pendant carboxyl groups. They may be ethoxylated and/or propoxylated at least at one terminal end. The carboxyl group can be a carboxylic acid group or an ester group, wherein the alkyl moiety of the ester group contains one to three carbon atoms. The carboxylated polyurethane resin can also be a copolymer of polyvinylpyrrolidone and a polyurethane, having a CTFA designation PVP/polycarbamyl polyglycol ester. Suitable carboxylated polyurethane resins are disclosed in EP-A-0619111 and US Patent No. 5,000,955.

Other suitable hydrophilic polyurethanes are disclosed in US Patent Nos. 3,822,238; 4,156,066; 4,156,067; 4,255,550; and 4,743,673.

Amphoteric hair styling polymers which can contain cationic groups derived from monomers such as t-butyl aminoethyl methacrylate as well as carboxyl groups derived from monomers such as acrylic acid or methacrylic acid can also be used in the present invention. One specific example of an amphoteric hair styling polymer is Amphomer® (Octylacrylamide/ acrylates/butylaminoethyl methacrylate copolymer) sold by the National Starch and Chemical Corporation.

Examples of nonionic hair styling polymers are homopolymers of N- vinylpyrrolidone and copolymers of N-vinylpyrrolidone with compatible nonionic monomers such as vinyl acetate. Nonionic polymers containing N- vinylpyrrolidone in various weight average molecular weights are available commercially from ISP Corporation - specific examples of such materials are homopolymers of N-vinylpyrrolidone having an average molecular weight of about 630,000 sold under the name PVP K-90 and are homopolymers of N-vinylpyrrolidone having an average molecular weight of about 1,000,000 sold under the name of PVP K-120.

Other suitable nonionic hair styling polymers are cross-linked silicone resins or gums. Specific examples include rigid silicone polymers such as those described in EP-A-0240350 and cross-linked silicone gums such as those described in WO 96/31188.

Examples of cationic hair styling polymers are copolymers of amino-functional acrylate monomers such as lower alkyl aminoalkyl acrylate, or methacrylate monomers such as dimethylaminoethyl methacrylate, with compatible monomers such as N-vinylpyrrolidone, vinyl caprolactam, alkyl methacrylates (such as methyl methacrylate and ethyl methacrylate) and alkyl acrylates (such as ethyl acrylate and n-butyl acrylate).

Specific examples of suitable cationic polymers are:
copolymers of N-vinylpyrrolidone and dimethylaminoethyl methacrylate, available from ISP Corporation as Copolymer 845, Copolymer 937 and Copolymer 958;
copolymers of N-vinylpyrrolidone and dimethylaminopropylacrylamide or methacrylamide, available from ISP Corporation as Styleze® CC10;
copolymers of N-vinylpyrrolidine and dimethylaminoethyl methacrylate;
copolymers of vinylcaprolactam, N-vinylpyrrolidone and dimethylaminoethylmethacrylate;
Polyquaternium-4 (a copolymer of diallyldimonium chloride and hydroxyethylcellulose);
Polyquaternium-11 (formed by the reaction of diethyl sulphate and a copolymer of vinyl pyrrolidone and dimethyl aminoethylmethacrylate), available from ISP as Gafquat® 734, 755 and 755N, and from BASF as Luviquat® PQ11;
Polyquaternium-16 (formed from methylvinylimidazolium chloride and vinylpyrrolidone), available from BASF as Luviquat® FC 370, FC 550, FC 905 and HM-552;
Polyquaternium-46 (prepared by the reaction of vinylcaprolactam and vinylpyrrolidone with methylvinylimidazolium methosulphate), available from BASF as Luviquat®Hold.

Examples of suitable naturally-derived polymers include shellac, alginates, gelatins, pectins, cellulose derivatives and chitosan or salts and derivatives thereof. Commercially available examples include Kytamer® (ex Amerchol) and Amaze® (ex National Starch).

Also suitable for use as optional components in the compositions of the invention are the ionic copolymers described in WO 93/03703, the polysiloxane-grafted polymers disclosed in WO 93/23446, the silicone-containing polycarboxylic acid copolymers described in WO 95/00106 or WO 95/32703, the thermoplastic elastomeric copolymers described in WO 95/01383, WO 95/06078, WO 95/06079 and WO 95/01384, the silicone grafted adhesive polymers disclosed in WO 95/04518 or WO 95/05800, the silicone macro-grafted copolymers taught in WO 96/21417, the silicone macromers of WO 96/32918, the adhesive polymers of WO 98/48770 or WO 98/48771 or WO 98/48772 or WO 98/48776, the graft polymers of WO 98/51261 and the grafted copolymers described in WO 98/51755.

With certain of the above-described polymers it may be necessary to neutralise some acidic groups to promote solubility/dispersibility. Examples of suitable neutralising agents include 2-amino-2- methyl-1, 3-propanediol (AMPD); 2-amino-2-ethyl-1,3-propanediol (AEPD); 2-amino-2-methyl-1-propanol (AMP); 2-amino-1-butanol (AB); monoethanolamine (MEA); diethanolamine (DEA); triethanolamine (TEA); monoisopropanolamine (MIPA); diisopropanol-amine (DIPA); triisopropanolamine (TIPA); and dimethyl stearamine (DMS). A long chain amine neutralising agent such as stearamidopropyl dimethylamine or lauramidopropyl dimethylamine may be employed, as is described in US 4,874,604. Also suitable are inorganic neutralisers, examples of which include sodium hydroxide, potassium hydroxide and borax. Mixtures of any of the above neutralising agents may be used. Amounts of the neutralising agents will range from about 0.001 to about 10% by weight of the total composition.
- sunscreening agents such as 2-ethylhexyl p-methoxycinnamate, 2-ethylhexyl N,N-dimethyl-p-aminobenzoate, p-aminobenzoic acid, 2-phenylbenzimidazole-5-sulfonic acid, octocrylene, oxybenzone, homomenthyl salicylate, octyl salicylate, 4,4'-methoxy-t-butyldibenzoylmethane, 4-isopropyl dibenzoylmethane, 3-benzylidene camphor, 3-(4-methylbenzylidene) camphor, titanium dioxide, zinc oxide, silica, iron oxide, and mixtures thereof.
- anti-dandruff actives such as zinc pyrithione, piroctone olamine, selenium disulphide, sulphur, coal tar, and the like.
- hair conditioning agents such as hydrocarbons, silicone fluids, and cationic materials. The hydrocarbons can be either straight or branched chain and can contain from about 10 to about 16, preferably from about 12 to about 16 carbon atoms. Examples of suitable hydrocarbons are decane, dodecane, tetradecane, tridecane, and mixtures thereof. Examples of suitable silicone conditioning agents useful herein can include either cyclic or linear polydimethylsiloxanes, phenyl and alkyl phenyl silicones, and silicone copolyols. Cationic conditioning agents useful herein can include quaternary ammonium salts, as mentioned hereinbefore as materials for incorporation into the aqueous phase, or the salts of fatty amines.
- emulsifiers for emulsifying the various carrier components of the compositions of the invention. Suitable emulsifier types include polyethylene glycol 20 sorbitan monolaurate (Polysorbate 20), polyethylene glycol 5 soya sterol, Steareth-20, Ceteareth-20, PPG-2 methyl glucose ether distearate, Ceteth-10, Polysorbate 80, cetyl phosphate, potassium cetyl phosphate, diethanolamine cetyl phosphate,Polysorbate 60, glyceryl stearate, PEG-100 stearate, and mixtures thereof. The emulsifiers can be used individually or as a mixture of two or more and can comprise from about 0.1% to about 10%, more preferably from about 1% to about 7%, and most preferably from about 1% to about 5%, by weight based on total weight of the composition.
- vitamins and derivatives thereof (e.g., ascorbic acid, vitamin E, tocopheryl acetate, retinoic acid, retinol, retinoids, and the like).
- cationic polymers (e.g., cationic guar gum derivatives such as guar hydroxypropyltrimonium chloride and hydroxypropyl guar hydroxypropyltrimonium chloride, available as the Jaguar® series from Rhone-Poulenc).
- preservatives, antioxidants, chelators and sequestrants; and aesthetic components such as fragrances, colourings, hair nutrients and essential oils.

The invention will now be illustrated by the following nonlimiting examples. In the examples and throughout this specification, all percentages are by weight based on total composition unless indicated otherwise.

The examples refer to the accompanying figures, which are illustrative and not limiting in any way, and in which:

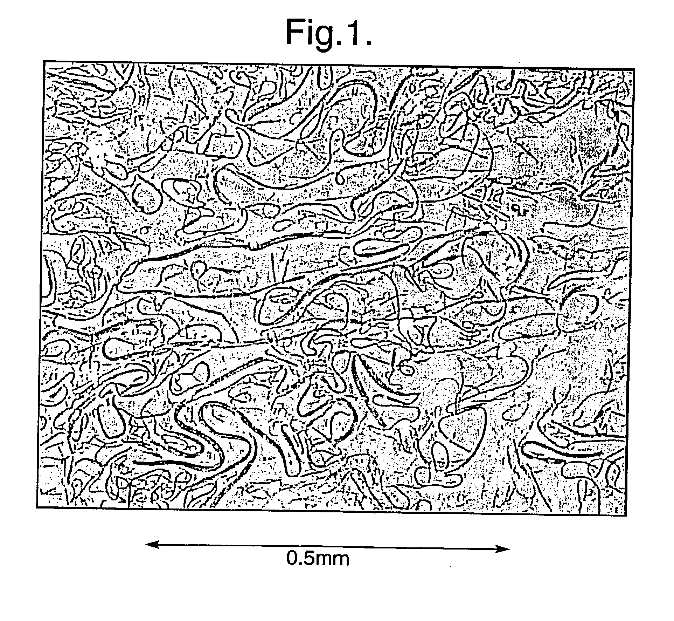

Figure 1 shows gelled fibres according to the invention;
Figure 2 shows gelled fibres encapsulating a silicone; and
Figure 3 shows the fibres of the invention deposited onto hair fibres.

### Examples

Particle shape analysis was carried out as follows. The fibre material was diluted with an aqueous solvent of the same ionic strength as present in the composition. This means that the fibre composite was diluted with its own solvent. For dilution, a certain amount of the fibre composite was added to a certain amount of the solvent while stirring with a paddle stirrer, or shaking in a flask rotator, to prepare a diluted sample for particle shape analysis that contained 1% w/w fibres. In the case of a gelled second phase, the type of salt to get the desired ionic strength was carefully chosen in order to melt the gel of the second phase on dilution ensuring that the particles of the first phase remain gelled. For instance, a gellan-kappa-carrageenan fibre composite was diluted with 0.4% w/w sodium chloride solution. As a next step, images of the sample were taken using conventional light microscopes under suitable lighting conditions, preferably phase contrast, and suitable magnification, preferably 1:40. Typically, images showed a number of separate fibres, very small apparently spherically shaped particles and larger entities that appeared to be associated fibres. Enough images were statistically taken such that 400 fibres were pictured as individual entities --meaning that they do not overlap with any other fibres. A particle was identified as a fibre when it showed a clear extension in one direction. If the length of a fibre was not pictured with its whole length, the pictured length of this fibre was measured and taken for aspect ratio calculation. The aspect ratio was calculated by dividing the length of the fibre by the width. The width of a fibre was measured at least once, most preferably several times, along the fibre length at equally spaced locations that were roughly a tenth of the image width apart, eg, a magnification of 1:40 corresponds to an image width of 800 µm, hence, the fibre width was measured every about 100 µm along the fibre length. Finally, the average of the measured values was taken as the width of the fibre.

### Example 1

**Two Stage Method for the Production of Gelled Fibres (from 2 biopolymers)**

### Stage 1: Making the Gelled Fibres

A fibre composite was prepared from a mixture of κ-carrageenan and CMC with the following material characteristics.

### κ-carrageenan:

- κ-carrageenan with low residual cations, in particular low potassium (Genuvisco X0909, ex Hercules Limited (UK)) was used.
- The typical composition on powder is:
   92.9 %w/w κ-carrageenan
   5.12 %w/w sodium ions
   0.18 %w/w potassium ions
   0.01 %w/w calcium ions
   0.01 % w/w magnesium ions
- The moisture content of the powder is 10 %w/w.
- The zero shear viscosity of a 1% w/w (on powder) aqueous solution at 20°C is 0.08 Pa.s ± 10%. carboxymethylcellulose (CMC) :
- A commercially available CMC (Blanose 7MF, ex CPKelco UK

Limited) was used.
- The ion content of the batch used was:
   7.6 %w/w sodium ions
   7X10⁻³ %w/w potassium ions
   11.8X10⁻³ %w/w calcium ions
   22.6X10⁻³ %w/w chloride ions
   5X10⁻³ %w/w sulfate ions
- The moisture content of the powder is 5 %w/w.
- The zero shear viscosity of a 1 %w/w (on powder) aqueous solution at 20°C is 0.06 Pa.s ± 10%.

60 g of the κ-carrageenan, 150 g of the CMC, and 15 g potassium chloride were mixed dry, and then added to 2775 g de-ionised water while stirring vigorously with a paddle stirrer. The mixture was then heated up to 95°C under continued stirring, and kept under these conditions until a liquid mixture of all ingredients had formed. The mixture was transferred into a jacketed and agitated pre-mix tank, which was pre-heated, to 95°C. The vessel could take a maximum of 5 l. In the pre-mix tank, the stirring of the mixture was continued, and after 15 minutes at 95°C the temperature was lowered to 80°C. Once this temperature was reached, the vessel was set under a hydrostatic pressure of 2.5 bar, and the outlet valve at the bottom of the pre-mix tank was opened to allow for the mixture to flow through a pipe with circular cross section which was jacketed for most of its length. The length and inner diameter of the pipe was 1.2 m and 8 mm respectively. The middle 0.5 m of the pipe was cooled by water jacket, the temperature of the water incoming at the further end of the jacket was 10°C. The water exited at the end of the jacket which was closer to the pre-mix tank. The product was collected under sterile conditions, and either directly used, dried, or stored in a freezer at -18°C. The shape characteristics of the product were not altered by the drying process, or the freezing process, and subsequent re-hydration or thawing respectively.

The hydrostatic pressure of 2.5 bar applied to the pre-mix tank corresponds to a wall shear stress of 417 Pa. This fibre composite material comprises a non-gelled second aqueous phase dominated by CMC. The particle phase comprises gelled κ-carrageenan fibres. The volume in the mixture occupied by the first, gelled, phase is about 20%.

### Stage 2: Rinsing and separation of κ-carrageenan fibres

The dispersion of κ-carrageenan fibres in CMC was diluted 1:1 by volume with a solution of 1% by weight CMC, 0.05% by weight sodium azide and 1% by weight KCl using a rotary mixer overnight. Then 1 part of the diluted fibre suspension was mixed with 3 parts by volume of a solution of 1% by weight KCl and 0.05% by weight sodium azide using a magnetic stirrer at a low setting at ambient temperature. The final solution was centrifuged at 5000rpm at 20°C for 15 minutes. After centrifugation, the supernatant was poured off, leaving the fibres as a pellet at the base of the tube.

### Example 2 (Comparative Example)

### Two Stage Method for the Production of Gelled Spheres (from 2 biopolymers)

### Stage 1: Making the Gelled Spheres

60 g of the κ-carrageenan used in Example 1, 150 g of the CMC used in Example 1, and 15 g potassium chloride were mixed dry, and then added to 2775 g de-ionised water while stirring vigorously with a paddle stirrer. The mixture was then heated up to 95°C under continued stirring, and kept under these conditions until a liquid mixture of all ingredients had formed. The mixture was then allowed to cool under quiescent conditions. The κ-carrageenan phase separated from the CMC as droplets and set to form gelled spheres on cooling.

### Stage 2: Rinsing and separation of κ-carrageenan spheres

This stage was carried out in exactly the same way as for the κ-carrageenan fibres (Example 1, stage 2).

### Example 3

### One Stage Method for the Production of Gelled Fibres

Gelled fibres of a polymer, such as κ-carrageenan, can be made directly into a surfactant. This can be achieved by mixing the carrageenan and the surfactant at high temperature (while the carrageenan is still molten), the mixture will then phase separate. As long as the phase volume of the carrageenan is less than the phase volume of the surfactant, then the carrageenan will form the droplet phase. Then, if low shear and fast cooling is applied, the carrageenan liquid droplets will elongate to form fibres which are then trapped into this morphology by gelation.

### Materials

κ-carrageenan - (Genuvisco X0909, ex Hercules Limited (UK), is about 90% pure kappa form, has low residual cations, in particular low potassium - 0.18%w/w).
SLES - Sodium Lauryl Ether Sulphate (26.2% active) (Empicol ESB 3/M; Albright and Wilson)
CAPB - Cocamidopropyl Betaine (30% active) (Tegobetaine CK - Goldschmidt)
Sodium Benzoate
Deionised Water

### Method:

Preparation of Biopolymer/Surfactant Mixture.
● Make a solution of κ-carrageenan (5.12% by weight) by sprinkling the carrageenan powder into the vortex of cold sodium benzoate solution (1% by weight).
● Heat to 95°C until the carrageenan has fully dissolved (solution becomes clear).
● Allow to cool to about 60°C (above the gelation temperature of carrageenan).
● Meanwhile, prepare the surfactant blend. This involves mixing 85.5g of SLES (26.2% by weight active) and 11.4g CAPB (30% by weight active) and heating to 60°C.
● Add 101.10g of carrageenan solution to the surfactant mixture and add 1% by weight (2g) NaCl. Note the mixture is now 200g, 50.55% by weight is carrageenan solution (at 5.12% by weight concentration), 48.45% by weight is surfactant (5.7% by weight of diluted CAPB and 42.75% by weight of diluted SLES) and 1% by weight is NaCl.
● Pour the mixtures into centrifuge tubes and centrifuge at 10 000rpm for 1 hour at 60°C, to obtain bulk phase separation. Then allow to cool. After cooling, the carrageenan-rich phase is a solid gel in the lower phase and the surfactant-rich phase is fluid in the upper phase.
● The two phases should then be manually separated into the carrageenan-rich phase and the surfactant rich phase and then re-mixed so that the carrageenan-rich phase is 30% of the total phase volume. This mixture can be heated and then cooled under required shear to produce the gelled fibres. Once the phase diagram has been constructed for any one set of conditions of carrageenan, temperature, salt and surfactant mix, then the initial mixture required for any final stated phase volume may be obtained. For this particular mixture, using this particular grade of carrageenan, 30% phase volume has been found to be the optimum phase volume for the formation of gelled fibres.

### Formation of Gelled Fibres

The Linkam microscope shearing stage was used in the formation of the gelled fibres. The Linkam shearing stage (Linkam CSS 450 microscope stage) is based on a parallel plate rheometer design, in conjunction with a slight hole to allow simultaneous microscope examination. The stage allows for the application of a range of shear on a sample under examination by light microscopy. Temperature, cooling/heating rate, shear and cell depth (gap) is controlled via software.

The gap was set at 2500µm and the sample placed onto the slide at 95°C. The molten liquid droplets of the κ-carrageenan-rich phase could be seen amongst the continuous surfactant-rich phase. A shear rate of 1s⁻¹ was employed and a cooling rate of 20°C/min. The images recorded during the fibre formation and gelation of the fibres and taken from the video show the elongation of the droplets into fibres. The carrageenan used in these images gels at around 40°C.

### Example 4

### Gelled Fibres as Delivery Agents - Entrapment of silicone/oil into gelled kappa-carrageenan (κ-carrageenan) particles

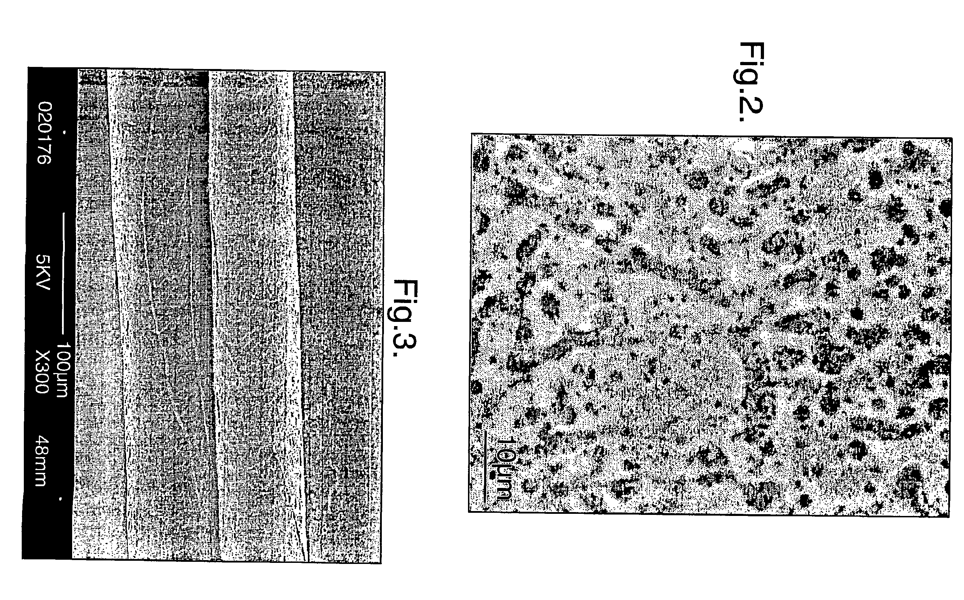

κ-carrageenan (2% by weight), carboxymethyl cellulose (CMC) (5% by weight) and potassium chloride (0.5% by weight) were mixed dry and then added to de-ionised water (to make up the weight of the mixture) while stirring vigorously with a paddle stirrer. The mixture was then heated up to 95°C under continued stirring and kept under these conditions until the polymers were fully hydrated. An aminosilicone emulsion (DC-7224 from Dow Corning, 500nm emulsion) was heated to 60°C and added to the polymer mixture (cooled to 60°C), these were mixed gently and then allowed to cool under quiescent conditions. The sample was then viewed under a light microscope and the results are shown in Figure 2.

It was found that the κ-carrageenan-rich phase had demixed from the continuous CMC-rich phase and that the aminosilicone had been preferentially entrapped within the gelled κ-carrageenan particles.

### Example 5

### Entanglement of gelled fibre into hair array

Two suspensions were prepared. The first was of κ-carrageenan spheres in CMC (prepared as in Example 4, but with no aminosilicone emulsion added) and the second was of κ-carrageenan fibres in CMC (prepared as in stage 1 of Example 1). The fibre suspension was then diluted with 1% by weight CMC/1% by weight KCl in a 1:1 weight ratio (to give about 10% by volume particle phase) and the spherical suspension was diluted with the same solution in a 2:1 weight ratio (spheres:solution) to give a 13% by volume particle phase.

The fibre suspension was rubbed through one side of a mannequin head with long hair and the spherical suspension was rubbed through the other side. Both sides were rinsed for the same period of time with the same flow rate of water.

Hair fibres were then taken from each side of the head and were examined using cryoSEM (Scanning Electron Microscopy). The results are shown in Figure 3. It could be seen that the fibres had entangled amongst the hair array whereas the spheres tended to be washed away.

This result suggests that gelled fibres containing an active can be used as a specific and targeted delivery agent.

### Example 6

### Sensory Evaluation

The following shampoo formulations containing gelled fibres (according to the invention, prepared as in Example 1), gelled spheres (first control, prepared as in Example 2) and neither fibres nor spheres (second control) were prepared. The fibres and spheres were added as 40% of the total composition weight. It was assumed that these fibres/spheres contained only κ-carrageenan gel and no CMC solution. The concentration of the κ-carrageenan within these fibres/spheres cannot be determined without the use of a phase diagram, but would be expected to be in the order of 8-10% by weight.

| | | FIBRES | CONTROL | SPHERES |
|---|---|---|---|---|
| COMPONENT | % BY WEIGHT ACTIVE IN COMPOSITION | W/W | W/W | W/W |
| SLES (28% wt active) | 14 | 50 | 50 | 50 |
| CAPB (30% wt active) | 2 | 6.67 | 6.67 | 6.67 |
| Fibres | 40 | 40 | - | - |
| Spheres | 40 | - | - | 40 |
| Carbopol (1% wt active) | 0.4 | - | 40 | - |
| Jaguar C13S (2% wt active) | 0.1 | 5.0 | 5.0 | 5.0 |
| NaCl (100% wt active) | 1 | 1 | 1 | 1 |

Where the component is not 100% active, the balance is water. Formulations containing slightly over 100% are based on the absolute amount of the components and can be recalculated as percentages by dividing the figures given by the total amount of the components.

Sensory testing on the three compositions was carried out. The attributes assessed were:
a) rate of foam formation
b) amount of foam generated
c) creaminess of foam

A wetted switch (254mm/7g) was pipetted with ~1ml of product along its length and was washed for 30seconds. This was repeated with a second switch with a different product and the subjects (13) were asked to assess the three above attributes.

The first assessment was the control shampoo compared to the shampoo comprising fibres:
Rate of formation: 7/13 thought the control shampoo foamed faster, 6/13 thought it was the fibre shampoo
Amount of foam: 3/13 thought the control shampoo Generated more foam, 6/13 thought it was the fibre shampoo, 4/13 thought both were the same
Creaminess: 3/13 thought the control shampoo was creamier, 10/13 thought it was the fibre shampoo

The second assessment was of the shampoo comprising spheres compared to the fibres shampoo:
Rate of formation: 7/13 thought the spheres foamed faster, 3/13 thought it was the fibres, 3/13 thought both were the same
Amount of foam: 7/13 thought the spheres generated more foam, 6/13 thought it was the fibres
Creaminess: 2/13 thought the spheres shampoo was creamier, 11/13 thought it was the fibres

The composition comprising the fibres therefore showed textural advantages, such as increased creaminess.

### Example 7

### Scalp Lotion

The following scalp lotion was prepared:

| Ingredient | Function | % by weight |
|---|---|---|
| Ethanol | Solvent | 40 |
| Glycerol | Humectant | 10 |
| Tween 20 | Emulsifier | 1 |
| Menthol | Cooling agent | 1 |
| Composite of κ-carrageenan gelled fibres in CMC | Rheology modifier | 10 (approx. 2% carrageenan gelled fibres, 8% CMC) |
| Water | Cosolvent | Balance |

The glycerol and Tween 20 were dissolved in water, ethanol was added and then mixed and then menthol was added and mixed. Gelled fibres (prepared as detailed in Stage 1, Example 1) were added to the resulting mixture to make up to 100%.

### Example 8

### Mousse

| Ingredient | Function | % by weight |
|---|---|---|
| Gafquat 755N | Styling polymer | 4 |
| Gafquat 734 | Styling polymer | 1.6 |
| Volpo CS50 | Surfactant | 0.3 |
| CAP40 | Propellant | 8 |
| Composite of κ-carrageenan gelled fibres in CMC | Structurant | 10 (Approx 2% gelled carrageenan fibres & 8% CMC) |
| Ethanol | Solvent | 8 |
| Water | | Balance |

Styling polymers, surfactant, propellant, solvent and water were mixed together to form 90% of the mix. Then the fibres (as prepared in Stage 1, Example 1) were added to the above formulation and mixed in to make up to 100%.

## Claims

1. Cosmetic or personal care composition comprising:
gelled particles, wherein the gelled particles comprise water, at least 50% by volume of the particles have an aspect ratio of at least 5 and a maximum width of 40µm,
and at least one cosmetically acceptable material selected from surfactants, hair conditioning agents, perfumes or fragrances and hair styling polymers.

2. Composition as claimed in claim 1, wherein at least 20% by volume of the particles have an aspect ratio of at least 10 and a maximum width of 20µm.

3. Composition as claimed in any one of claims 1 to 2,
wherein at least 5% by volume of the particles have an aspect ratio of at least 50 and a maximum width of 2µm.

4. Composition as claimed in any one of claims 1 to 3,
wherein greater than 90% of the particles have a minimum width of 0.5µm.

5. Composition as claimed in any one of claims 1 to 4,
wherein the gelled particles comprise a gelling polymer.

6. Composition as claimed in claim 5, wherein the gelling polymer is selected from kappa-carrageenan, gellan, gelatin and alginate.

7. Composition as claimed in claim 6, wherein the gelling polymer is kappa-carrageenan.

8. Composition as claimed in any one of claims 1 to 7,
wherein the gelled particles are present in the composition in an amount of from 0.1% to 60% by volume of the composition.

9. Composition as claimed in any ohe of claims 1 to 8, which is a hair treatment composition.

10. Composition as claimed in claim 9, which is a shampoo composition comprising from 1% to 50% by weight of one or more surfactants.

11. Composition as claimed in claim 9, which is a hair conditioning composition comprising from 0.1% to 30% by weight of one or more hair conditioning agents.

12. Composition as claimed in claim 9, which is a hair styling composition comprising from 0.1% to 10% by weight of one or more hair styling polymers.

13. Composition as claimed in any one of claims 1 to 12,
wherein the gelled particles comprise a hair benefit agent.

14. Composition as claimed in claim 13, wherein the hair benefit agent is selected from hair and skin conditioners, hair and skin cleansers, hair fixatives, hair dyes, hair growth promoters, permanent wave compounds, hair relaxers, hair straighteners, antibacterial compounds, antifungal compounds and anti-inflammatory compounds.

15. Method of treating hair comprising applying to the hair a composition comprising gelled particles comprising water, wherein at least 50% by volume of the particles have an aspect ratio of at least 5 and a maximum width of 40 µm.

16. Use of gelled particles comprising water wherein at least 50% by volume of the particles have an aspect ratio of at least 5 and a maximum width of 40 µm for providing textural properties to a cosmetic or personal care composition.

17. Process for producing a cosmetic or personal care composition according to any one of claims 1 to 14 which comprises forming the gelled particles and then bringing the gelled particles into contact with the at least one cosmetically acceptable material.

## Patentansprüche

1. Kosmetische oder Körperpflegezusammensetzung, umfassend gelierte Partikel, wobei die gelierten Partikel Wasser umfassen, wenigstens 50 Vol.-% der Partikel ein Dimensionsverhältnis von wenigstens 5 und eine maximale Breite von 40 µm haben, und wenigstens ein kosmetisch verträgliches Material, ausgewählt aus oberflächenaktiven Mitteln, Haarkonditionierungsmitteln, Parfüms oder Duftmaterialien und Haarstyling-Polymeren.

2. Zusammensetzung, wie sie in Anspruch 1 beansprucht wird, wobei wenigstens 20 Vol.-% der Partikel ein Dimensionsverhältnis von wenigstens 10 und eine maximale Breite von 20 µm haben.

3. Zusammensetzung, wie sie in einem der Ansprüche 1 bis 2 beansprucht wird, wobei wenigstens 5 Vol.-% der Partikel ein Dimensionsverhältnis von wenigstens 50 und eine maximale Breite von 2 µm haben.

4. Zusammensetzung, wie sie in einem der Ansprüche 1 bis 3 beansprucht wird, wobei mehr als 90 % der Partikel eine minimale Breite von 0,5 µm haben.

5. Zusammensetzung, wie sie in einem der Ansprüche 1 bis 4 beansprucht wird, wobei die gelierten Partikel ein gelierendes Polymer umfassen.

6. Zusammensetzung, wie sie in Anspruch 5 beansprucht wird, wobei das gelierende Polymer aus kappa-Carrageenan, Gelan, Gelatine und Alginat ausgewählt ist.

7. Zusammensetzung, wie sie in Anspruch 6 beansprucht wird, wobei das gelierende Polymer kappa-Carrageenan ist.

8. Zusammensetzung, wie sie in einem der Ansprüche 1 bis 7 beansprucht wird, wobei die gelierten Partikel in der Zusammensetzung in einer Menge von 0,1 bis 60 Vol.-% der Zusammensetzung vorliegen.

9. Zusammensetzung, wie sie in einem der Ansprüche 1 bis 8 beansprucht wird, welche eine Haarbehandlungszusammensetzung ist.

10. Zusammensetzung, wie sie in Anspruch 9 beansprucht wird, welche eine ShampooZusammensetzung ist, die 1 bis 50 Gew.-% eines oder mehrerer oberflächenaktiver Mittel umfasst.

11. Zusammensetzung, wie sie in Anspruch 9 beansprucht wird, die eine Haarkonditionierungs-Zusammensetzung ist, die 0,1 bis 30 Gew.-% eines oder mehrerer Haarkonditionierungsmittel umfasst.

12. Zusammensetzung, wie sie in Anspruch 9 beansprucht wird, die eine Haarstyling-Zusammensetzung ist, die 0,1 bis 10 Gew.-% eines oder mehrerer Haarstyling-Polymere umfasst.

13. Zusammensetzung, wie sie in einem der Ansprüche 1 bis 12 beansprucht wird, wobei die gelierten Partikel einen Wirkstoff für das Haar umfassen.

14. Zusammensetzung, wie sie in Anspruch 13 beansprucht wird, wobei der Wirkstoff für das Haar aus Haar- und Hautkonditionierungsmitteln, Haar- und Hautreinigungsmitteln, Haarfestigern, Haarfarben, Haarwachstums-Promotoren, Dauerwellenverbindungen, Haar-relaxierenden Mitteln, Haarglättungsmitteln, antibakteriellen Verbindungen, Antipilzverbindungnen und entzündungshemmenden Verbindungen ausgewählt ist.

15. Verfahren zur Behandlung von Haar, umfassend Auftragen einer Zusammensetzung, die gelierte Partikel umfasst, die Wasser umfassen, wobei wenigstens 50 Vol.-% der Partikel ein Dimensionsverhältnis von wenigstens 5 und eine maximale Breite von 40 µm haben, auf das Haar.

16. Verwendung von gelierten Partikeln, die Wasser umfassen, wobei wenigstens 50 Vol.-% der Partikel ein Dimensionsverhältnis von wenigstens 5 und eine maximale Breite von 40 µm haben, zur Verleihung von Textureigenschaften für eine kosmetische oder Körperpflegezusammensetzung.

17. Verfahren zur Herstellung einer kosmetischen oder Körperpflegezusammensetzung gemäß einem der Ansprüche 1 bis 14, das das Bilden der gelierten Partikel und danach In-Kontakt-Bringen der gelierten Partikel mit dem wenigstens einen kosmetisch verträglichen Material umfasst.

## Revendications

1. Composition cosmétique ou d'hygiène corporelle comprenant : des particules gélifiées, dans laquelle les particules gélifiées comprennent de l'eau, au moins 50 % en volume des particules ayant un rapport d'aspect d'au moins 5 et une largeur maximale de 40 µm, et au moins un matériau cosmétiquement acceptable choisi parmi les tensioactifs, les agents de conditionnement des cheveux, les parfums ou les fragrances et les polymères de coiffage des cheveux.

2. Composition selon la revendication 1, dans laquelle au moins 20 % en volume des particules ont un rapport d'aspect d'au moins 10 et une largeur maximale de 20 µm.

3. Composition selon l'une quelconque des revendications 1 à 2, dans laquelle au moins 5 % en volume des particules ont un rapport d'aspect d'au moins 50 et une largeur maximale de 2 µm.

4. Composition selon l'une quelconque des revendications 1 à 3, dans laquelle plus de 90 % des particules ont une largeur minimale de 0,5 µm.

5. Composition selon l'une quelconque des revendications 1 à 4, dans laquelle les particules gélifiées comprennent un polymère gélifiant.

6. Composition selon la revendication 5, dans laquelle le polymère gélifiant est choisi parmi la carraghénane kappa, la gomme gellane, la gélatine et l'alginate.

7. Composition selon la revendication 6, dans laquelle le polymère gélifiant est la carraghénane kappa.

8. Composition selon l'une quelconque des revendications 1 à 7, dans laquelle les particules gélifiées sont présentes dans la composition en une quantité de 0,1 à 60 % en volume de la composition.

9. Composition selon l'une quelconque des revendications 1 à 8, qui est une composition de traitement des cheveux.

10. Composition selon la revendication 9, qui est une composition de shampooing comprenant de 1 à 50 % en poids d'un ou de plusieurs tensioactifs.

11. Composition selon la revendication 9, qui est une composition de conditionnement des cheveux comprenant de 0,1 1 à 30 % en poids d'un ou de plusieurs agents de conditionnement des cheveux.

12. Composition selon la revendication 9, qui est une composition de coiffage des cheveux comprenant de 0,1 à 10 % en poids d'un ou de plusieurs polymères de coiffage des cheveux.

13. Composition selon l'une quelconque des revendications 1 à 12, dans laquelle les particules gélifiées comprennent un agent bénéfique pour les cheveux.

14. Composition selon la revendication 13, dans laquelle l'agent bénéfique pour les cheveux est choisi parmi les conditionneurs de cheveux et de peau, les lotions nettoyantes pour cheveux et peau, les fixateurs de cheveux, les colorants pour cheveux, les agents favorisant la pousse des cheveux, les composés pour permanentes, les produits défrisants, les produits lissants, les composés antibactériens, les composés antifongiques et les composés anti-inflammatoires.

15. Procédé de traitement des cheveux comprenant l'application aux cheveux d'une composition comprenant des particules gélifiées comprenant de l'eau, au moins 50 % en volume des particules ayant un rapport d'aspect d'au moins 5 et une largeur maximale de 40 µm.

16. Utilisation de particules gélifiées comprenant de l'eau, au moins 50 % en volume des particules ayant un rapport d'aspect d'au moins 5 et une largeur maximale de 40 µm pour conférer des propriétés texturales à une composition cosmétique ou d'hygiène corporelle.

17. Procédé de production d'une composition cosmétique ou d'hygiène corporelle selon l'une quelconque des revendications 1 à 14 qui comprend la formation des particules gélifiées, puis la mise en contact des particules gélifiées avec ledit au moins matériau cosmétiquement acceptable.
